# EUROPEAN PATENT SPECIFICATION

(11) **EP 3 162 336 B1**
(45) Date of publication and mention of the grant of the patent: **17.10.2018**
(21) Application number: 15815401.3
(22) Date of filing: 20.04.2015
(51) Int. Cl.: A61F 13/15, A61F 13/511, A61F 13/514, A61F 13/53, A61F 13/47, A61F 13/513

(54) **ABSORBENT ARTICLE**
SAUGFÄHIGER ARTIKEL
ARTICLE ABSORBANT

(30) Priority: 30.06.2014 JP 2014135425
(43) Date of publication of application: 03.05.2017
(73) Proprietor: Unicharm Corporation, Ehime 799-0111 (JP)
(72) Inventor: NOMOTO, Takashi, Kanonji-shi Kagawa 769-1602 (JP)
(74) Representative: Dolleymores
(86) International application number: PCT/JP2015/061940
(87) International publication number: WO 2016/002312

(56) References cited:
- WO-A1-95/12488
- WO-A1-97/01996
- WO-A1-2005/004770
- JP-A- H09 504 488
- JP-A- 2004 532 758
- JP-A- 2005 040 235
- JP-B2- 3 998 712
- US-A- 4 834 741
- US-A- 6 059 764

## Description

### {Technical Field}

The present invention relates to absorbent articles suitable to be used, for example, as sanitary napkins, urine-absorbing pads and panty-liners.

### {Background}

Pad-like absorbent articles adapted to be attached to the interior surface in a crotch region of a pant-type article such as sanitary pants is known. Similar articles extensible in a length direction of the article and adapted to be put on the wearer so as to extend in a front-back direction of the wearer are also known.

For example, an absorbent article disclosed in Patent Literature 1 has a longitudinal direction and a transverse direction being orthogonal to each other and includes a liquid-permeable topsheet, a backsheet, an absorbent body interposed between these two sheets, both end portions opposed to each other in the longitudinal direction and both lateral portions opposed to each other in the transverse direction wherein top- and backsheets have an extensibility in the longitudinal direction, the topsheet has a contractility in the longitudinal direction from a its stretched state property and the absorbent body is formed with slits extending in the transverse direction from the both lateral portions.

### {Citation List}

### {Patent Literature}

{PTL 1}: 2009-153835 A. Further prior art arrangements are known from WO97/01996, US6059764, US4834741, WO95/12488 and WO2005/004770.

### {Summary}

### {Technical Problem}

In conventional sanitary napkins, an extensibility of the topsheet and the backsheet in the longitudinal direction is uniform. When such napkin is subjected to forces opposite in the longitudinal direction, respective portions of the top- and backsheets facing the regions of the absorbent body in which the slits are present are stretched but respective portions of the top- and backsheets facing the regions of the absorbent body in which none of the slits are present are irregularly deformed and, in consequence, the tensile force insufficiently acts on the napkin to stretch the napkin at a desired degree.

An object of the present invention is to provide an absorbent article adapted to be sufficiently stretched when the absorbent article is subjected to tensile forces opposite in the longitudinal direction.

### {Solution to Problem}

To achieve the object set forth above, the present invention comprises a pad-shaped absorbent article as recited in claim 1.

### {Advantageous Effects of Invention}

The absorbent article according to one or more of the present invention is arranged so that at least one of the slits of the absorbent body may overlap with the first regions and, consequently, the first regions are elastically stretched as the slits of the absorbent body are opened. Further, one of the sheets has the first regions extending in the transverse direction across the absorbent body to express the elastic extensibility and the second regions having the elastic extensibility in the longitudinal direction lower than that of the first regions. With such arrangement, it is possible to make a deformation in the longitudinal direction significant in the first regions and to make a deformation in the second regions insignificant. In this way, it is possible to subject the article to tensile forces opposite in the longitudinal direction to stretch the article sufficiently in the longitudinal direction.

### {Brief Description of Drawings}

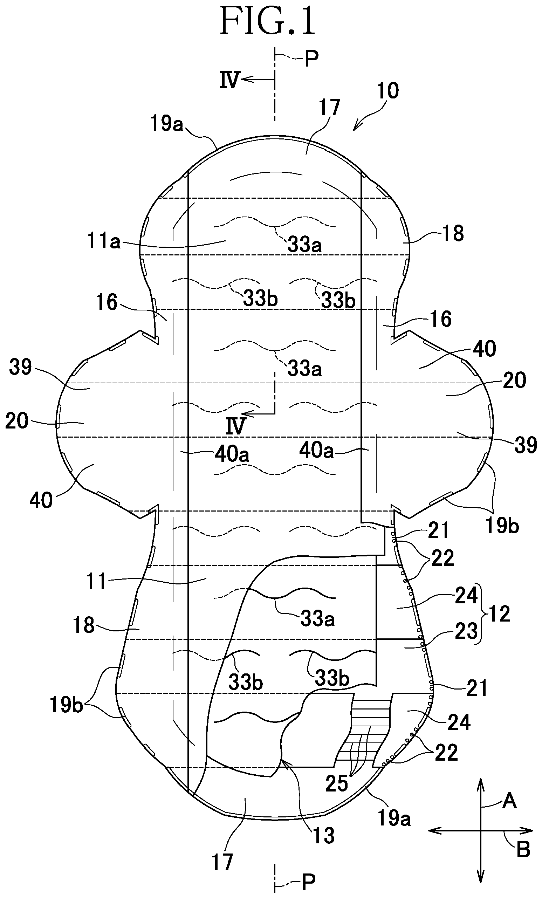

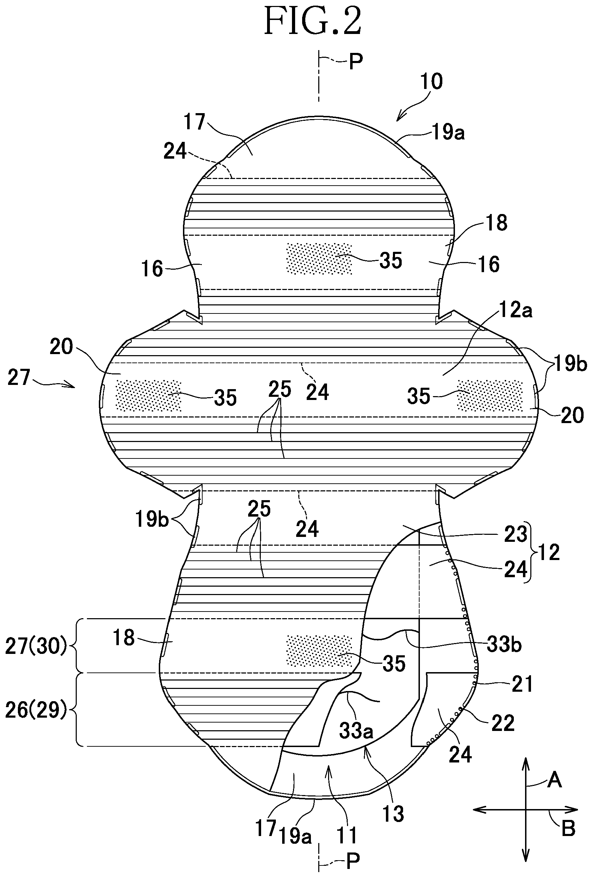

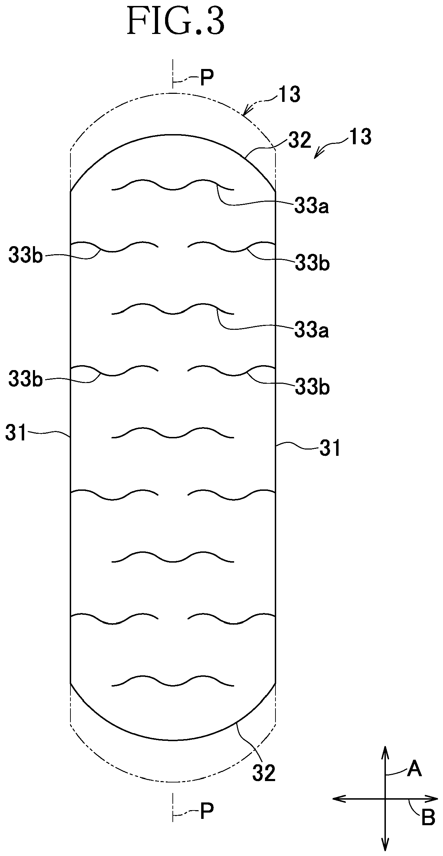

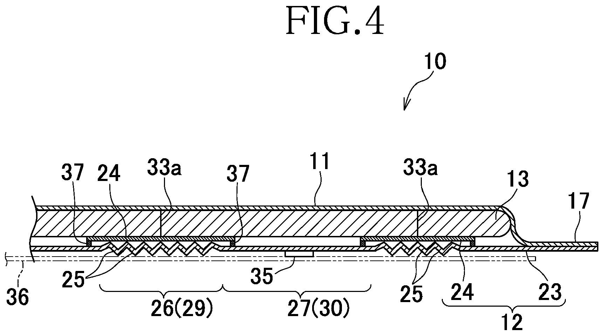

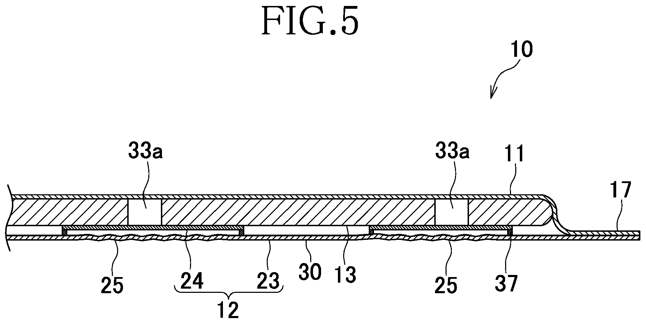

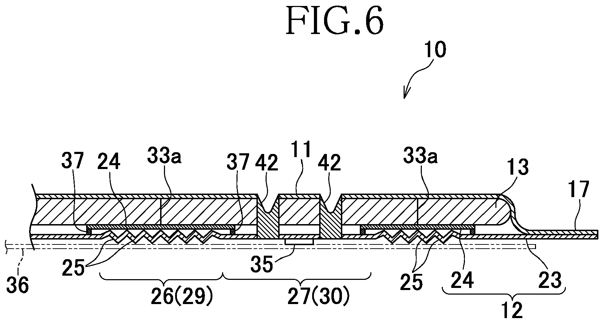

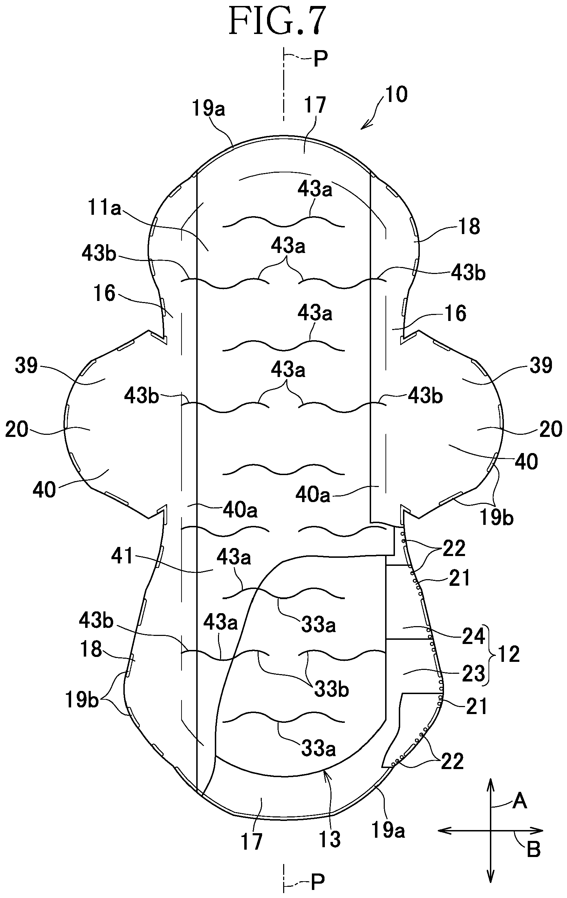

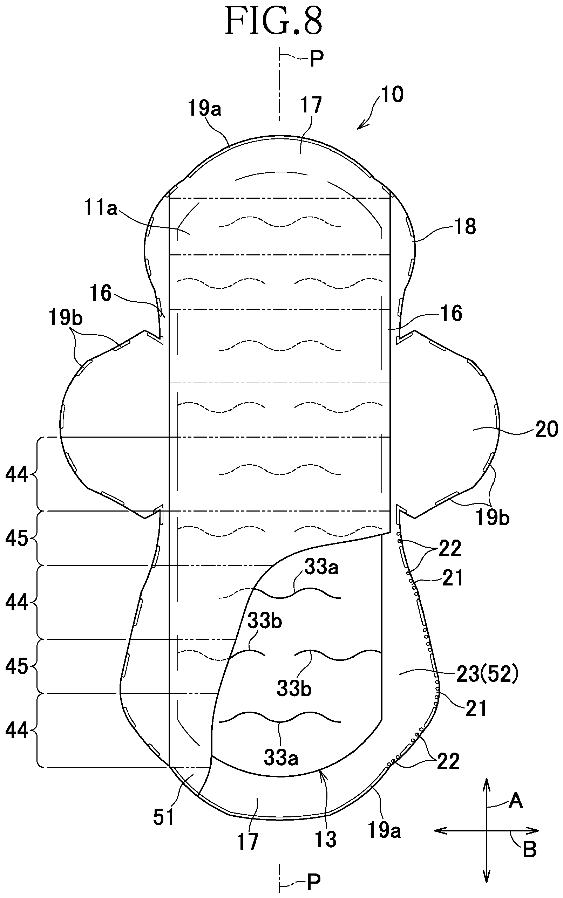

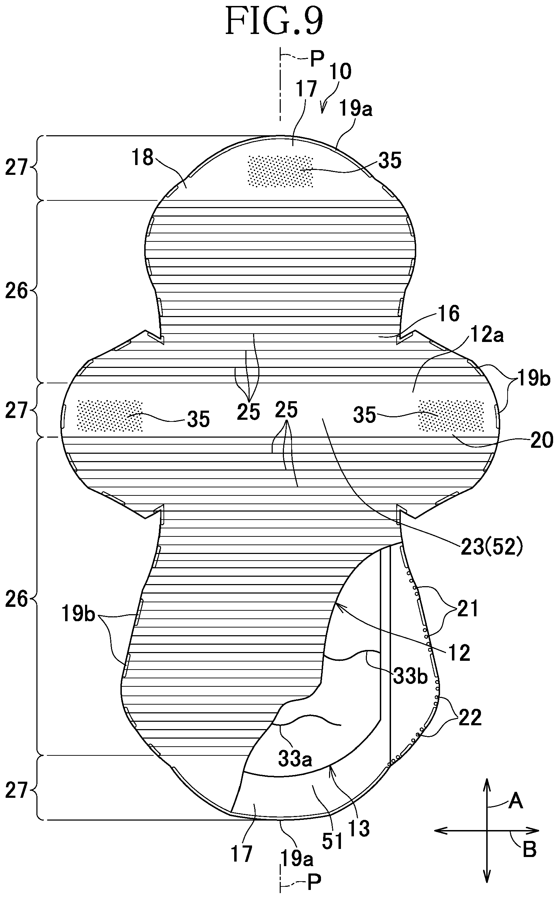

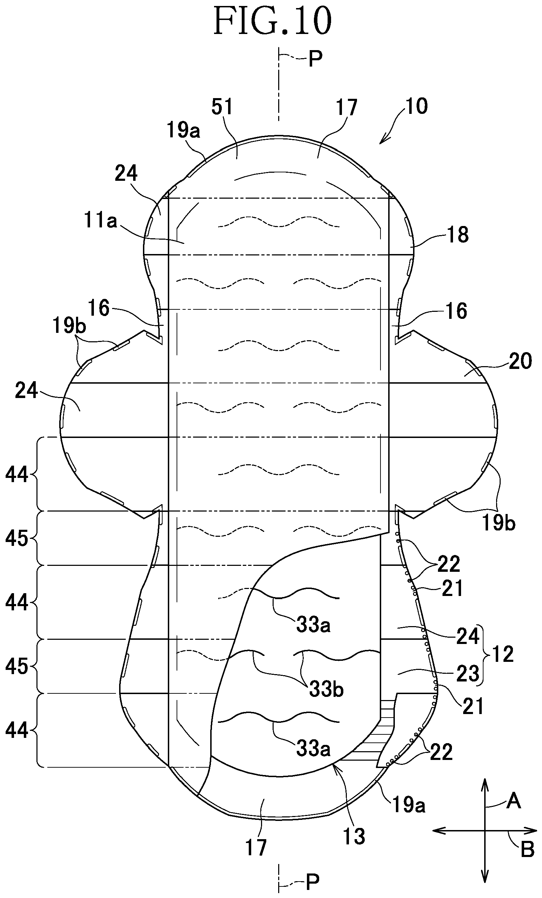

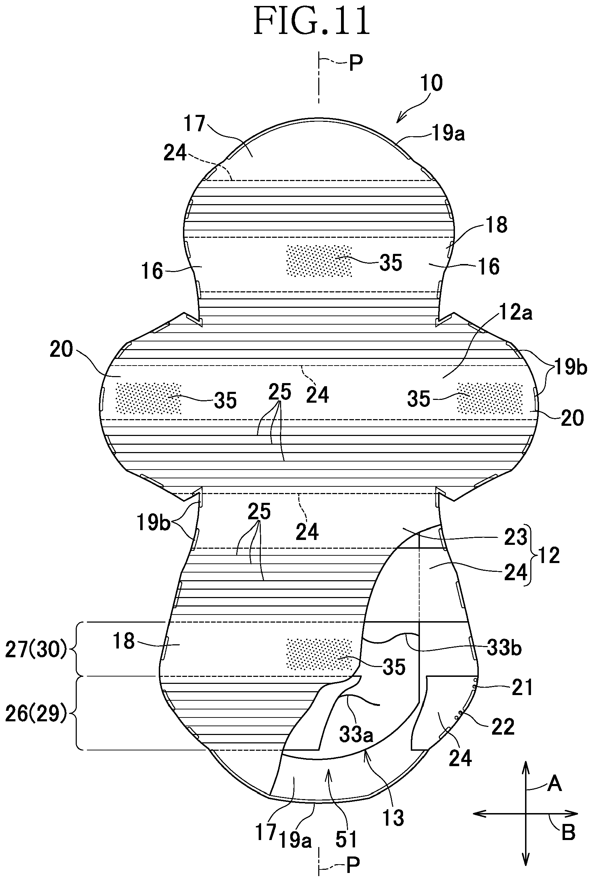

The drawings illustrate specific embodiments of the present invention includes optional and embodiments as well as essential features of the invention.
{Fig. 1} Fig. 1 is a partially cutaway plan view illustrating a skin-facing surface of a sanitary napkin as one example of absorbent articles according to a first embodiment of the present invention.
{Fig. 2} Fig. 2 is a partially cutaway plan view illustrating a garment-facing surface of a napkin illustrated in Fig. 1.
{Fig. 3} Fig. 3 is a plan view of an absorbent body.
{Fig. 4} Fig. 4 is a sectional view taken along line IV-IV in Fig. 1.
{Fig. 5} Fig. 5 is a view similar to Fig. 4, illustrating the napkin in its stretched state.
{Fig. 6} Fig. 6 is a view similar to Fig. 4, illustrating the napkin according to a second embodiment.
{Fig. 7} Fig. 7 is a view similar to Fig. 1, illustrating the napkin according to a third embodiment.
{Fig. 8} Fig. 8 is a partially cutaway plan view illustrating the skin-facing surface of the napkin according to a first example with a leakage-barrier sheet removed.
{Fig. 9} Fig. 9 is a view similar to Fig. 2, illustrating the napkin in Fig. 8.
{Fig. 10} Fig. 10 is a partially cutaway plan view illustrating the skin-facing surface of the napkin according to a fourth embodiment with the leakage-barrier sheet removed.
{Fig. 11} Fig. 11 is a view similar to Fig. 2, illustrating the napkin in Fig. 10.

### {Description of Embodiments}

The embodiments described below relate to a pad-shaped absorbent article illustrated in Figs. 1 through 11, including both optional and preferred features as well as those features which are essential features of the present invention.

### <First Embodiment>

Referring to Figs. 1 through 4, a sanitary napkin 10 as an example of an absorbent article according to the present invention has a longitudinal direction A and a transverse direction B, a center line P extending in the longitudinal direction A, and includes a topsheet 11 defining a surface facing the (not shown) skin of wearer (a skin-facing surface) 11a, a backsheet 12a (one of sheets in the first embodiment) defining surface facing the wearer's garment (a garment-facing surface) 12a (see Fig. 2) and an absorbent body 13 disposed between these both sheets 11, 12.

The napkin 10 has opposite longitudinal sides 16 and opposite lateral ends 17. The opposite longitudinal sides 16 are respectively formed with wings 20 projecting therefrom in the transverse direction B. The opposite longitudinal sides 16 cooperate with the opposite lateral ends 17 to define a peripheral portion 18 of the napkin 10. The opposite lateral ends 17 have continuous compressed recesses 19a in which the topsheet 11 and the backsheet 12 overlapping with each other are joined together. The opposite longitudinal sides 16 and the associated wings 20 respectively have continually compressed recesses 19b in which the backsheet 12 and side corner sheets 39 being stretchable in the longitudinal direction A and overlapping with the backsheet 12 are joined together. The backsheet 12 and the side corner sheets 39 are joined together also in edges 21 defined between each pair of the compressed recesses 19b adjacent in the longitudinal direction A with adhesive, preferably with elastically stretchable adhesive 22.

Referring again to Fig. 1, a region of the topsheet 11 covering at least the absorbing surface of the absorbent body 13 has a liquid-permeability. The topsheet 11 is formed of, for example, liquid-permeable fibrous nonwoven fabric or porous plastic films having a stretchability, more preferably an elastic stretchability in the longitudinal direction A. Such topsheet 11 formed of fibrous nonwoven fabrics, for example, a spunbonded nonwoven fabric of which the constituent fibers have a relatively high orientation degree in the transverse direction B. For such topsheet 11, the orientation degree of the constituent fibers is relatively high in a machine direction in a production step of the nonwoven fabric used as the material for the topsheet 11. If such nonwoven fabric obtained in this is arranged in the transverse direction B in the assembling step of constituent elements for the napkin, a relatively high orientation degree of the constituent fibers will be ensured in the transverse direction B. As the other materials for the topsheet 11, nonwoven fabrics containing crimped thermoplastic synthetic fibers or the nonwoven fabrics containing elastic fiber such as polyurethane fibers may be used. Also here, the constituent fiber in the napkin is preferably arranged so as to be oriented in the transverse direction B.

In the description of the present invention, as used here, the term "the sheets such as the topsheet 11 are extensible" means that, when a test piece cut out from the sheet so as to have a dimension of 50 mm in the longitudinal direction A and 25 mm in the transverse direction B is set in a tensile tester with respective 10 mm ends in the longitudinal direction A being chucked and the free length of 30 mm in the longitudinal direction A is pulled at a rate of 100 mm/min so that a stretching force (load) of 30 mN in the longitudinal direction A may be applied to the test piece per the dimension of 25 mm in the transverse direction B, a stretch ratio at least in a range of 1.03 to 1.10 (on the assumption that a length dimension in the longitudinal direction A of the sheet is 1.00 when no load is applied thereto, a length in the longitudinal direction A of the sheet is at least in a range of 1.03 to 1.10). As a matter of course, the stretch ratio of the sheet may be higher than 1.10. In the description of the present invention, as used herein, the term "the napkin 10 is extensible" means that, when the opposite lateral ends 17 of the napkin 10 in a range of 10 to 20 mm are gripped by the chucks of the tensile tester and the test piece is subjected to a stretching force of 700 mN/width dimension of 25 mm, the sanitary napkin 10 is stretched between the chucks at a stretch ratio at least in a range of 1.03 to 1.10 without being damaged. The numerical range mentioned above is ensured even when, for example, the topsheet 11 has not elastic extensibility and only the backsheet 12 has an elastic extensibility and, on the assumption that both the topsheet 11 and the backsheet 12 have an elastic extensibility, a stretch ratio of the napkin 10 may be even higher than 1.10. The stretch ratio of the napkin 10 is measured on the napkin 10 having the side corner sheets 39 removed therefrom. The sanitary napkin 10 is stretched preferably at a rate of 100 mm/min. As used herein, the term "the sheet has an elastic extensibility" means a behavior such that, when the sheet is subjected to a stretching force until the sheet is stretched in the longitudinal direction A to a length 2.00 times of the initial length and immediately thereafter the sheet is released from the stretching force, the sheet regains its length 1.50 times or less of the initial length thereof. If it is impossible for the sheet to regain its length 1.50 times or less of the initial length thereof, it is meant that such sheet has an inelastic extensibility. As used herein, the term "such napkin 10 is subjected to tensile forces opposite to each other in the longitudinal direction A" means that the opposite lateral ends 17 of the napkin 10 are respectively grasped and pulled so as to be distanced from each other in the longitudinal direction A or the one ends 17 is fixed and the other end 17 is pulled so as to be distanced from the one end 17.

Referring to Fig. 2, the backsheet 12 includes a non-extensible liquid-impermeable, more preferably a breathable but liquid-impermeable first sheet 23 located on the side of the garment-facing surface 12a and a plurality of elongate elastic second sheets 24 extending across the absorbent body 13. These second sheets 24 are arranged at predetermined intervals in the longitudinal direction A. Though not illustrated, the backsheet may be formed of a single fibrous nonwoven fabric having an elastic extensibility or of laminated two or more sheets.

The first sheet 23, when this is liquid-impermeable, may be formed of plastic films, for example, plastic films such as polyethylene resin films. The first sheet 23, when this is breathable and liquid-impermeable, may be formed of plastic films such as oriented polyethylene resin films containing fine particles of inorganic material, for example, calcium carbonate or barium sulfate as fillers. The first sheet 23 is formed with a plurality of pleats 25 extending across the absorbent body 13 from one of the longitudinal side 16 to the other longitudinal side 16. A plurality of pleats 25 are adjacent one to another in the longitudinal direction A to define each multi-pleats region 26. Between each pair of the multi-pleats regions, a pleat-free region 27 is defined. In such backsheet 12, the nonstretchable first sheet 23 defining the multi-pleats regions 26 cooperates with the elastically extensible second sheets 24 to define first regions 29 expressing an elastic extensibility and, in the respective pleat-free regions 27, second regions 30 in which the elastic extensibility in the longitudinal direction A is lower than that in the first regions 29. A cross-sectional shape of the pleats 25 taken in the longitudinal direction A is corrugated as exemplarily illustrated in Fig. 4 and, in view of this, these pleats 25 in the opposite longitudinal sides 16 are preferably in flatten state not only in the compressed recesses 19a, 19b but also in the region between each pair of the adjacent compressed recesses to prevent these pleats 25 in the opposite longitudinal sides 16 from irritating the wearer's skin.

The second sheets 24 are formed of a plastic film having a rubber elasticity, for example, polyurethane resin films and laid between the first sheet 23 and the absorbent body 13 so as to overlap the multi-pleats regions 26, preferably so as to cover the entirety of the multi-pleats regions 26. These second sheets 24 are joined, in their elastically relaxed condition, to the pleat-free regions 27 of the first sheet 23 through, for example, hot melt adhesive 37 (see Fig. 4) distributed at intervals at least in one of the longitudinal direction A and the transverse direction B.

The respective second sheets 24 are distanced one from another in the longitudinal direction A to avoid a possibility that the pleat-free region 27 defined between each pair of the adjacent multi-pleats regions 26 might be entirely covered with the respective second sheets 24. With such arrangement, the respective pleat-free regions 27 directly face the absorbent body 13 not through the intermediary of the second sheets 24. The pleat-free regions 27 are respectively provided on the garment-facing surfaces 12a with attachment regions 35 formed of, for example, pressure-sensitive adhesives so that the pleat-free regions 27 may be attached to the garment in a peelable manner. These attachment regions 35 are protectively covered with respective separators 36 indicated by imaginary lines during a period elapsing from production of the napkin to actual use thereof. A plurality of the attachment regions 35 formed on the napkin are sufficiently distanced from each other in the longitudinal direction A to prevent the extensibility of the napkin in the longitudinal direction A from being inhibited.

The pleat-free regions 27 in the respective wings 20 also are provided with the attachment regions 35. In this manner, all the attachment regions 35 are arranged unexceptionally in the pleat-free regions 27 of the napkin 10. In this regard, when the napkin 10 is put on the wearer' body, the wings 20 are folded back so as to overlap the exterior surface of the garment' s crotch region and attached to the garments through the intermediary of the respective attachment regions 35. If the wings 20 are provided with the multi-pleats regions 26, there is a likelihood that these multi-pleats regions 26 might make it difficult to fold the wings onto the exterior surface of the garments when the napkin 10 is attached to the clothes . To avoid such likelihood, the wings are preferably formed with none of the multi-pleats regions, though not illustrated.

Referring to Fig. 3, the absorbent body 13 may be provided in the form of an assembly of hydrophilic fibers and/or water-absorbent fibers compressed and shaped, a mixture of superabsorbent polymer particles and hydrophilic fibers compressed and shaped or such compressed and shaped assembly or mixture wrapped with tissue paper or liquid-permeable fibrous nonwoven fabrics, containing a binder such as water-soluble binder as the case may be. As materials for the hydrophilic fibers and/or water-absorbent fibers, it is possible to use rayon fibers, staple of acetate fibers, fluff pulp, wood pulp or synthetic pulp. The absorbent body 13 has opposite longitudinal sides 31 and opposite longitudinal ends 32.

The absorbent body 13 has a plurality of central region slits 33a and lateral region slits 33b. The respective central region slits 33a are arranged in the central region of the absorbent body as viewed in the transverse direction B so as to extend across the center line P, thereby dividing the absorbent body 13 partially in the longitudinal direction A. In other words, the absorbent body 13 is continuous in the longitudinal direction A on both lateral side regions in the transverse direction B of the central region slits 33a since none of the central region slits 33a extends to the opposite longitudinal sides 31. The lateral region slits 33b are arranged so as to extend from the opposite longitudinal sides 31 toward the central line P in the transverse direction B. In other words, the absorbent body 13 is continuous in the longitudinal direction A on the sides of the lateral region slits 33b close to the central line P since none of the lateral region slits 33b extends to the central line P. Preferably, these central region slits 33a and lateral region slits 33b are formed symmetrically about the center line P and extend through the absorbent body 13 in a thickness direction. While each of the slits 33a, 33b is preferably shaped in a curved line such as a waved line, a linear each of the slits 33a, 33b may be a liner slit. However, in comparison with the linear slits, the slits 33a, 33b shaped in the wave-like curved lines more effectively alleviate an uncomfortable feeling which the wearer might experience when the absorbent body 13 is inelastically stretched in the longitudinal direction A. For the absorbent body 13 having such slits 33a, 33b, the inelastic extensibility in the longitudinal direction A is relatively high in the regions in which the slits 33a, 33b are present and the inelastic extensibility in the longitudinal direction A is relatively low in the region in which the slits 33a, 33b are not present. While, in this napkin 10, the absorbent body 13 and the backsheet 12 are arranged and joined together so that all the central region slits 33a may overlap with the first regions 29, it is also possible to arrange the absorbent body 13 and the backsheet 12 and to join them together so that at least one of the slits 33a and 33b overlap with the first regions 29.

The absorbent body 13 is joined to the topsheet 11 and/or the backsheet layer 12, for example, with hot melt adhesive (not shown) and the central region slits 33a and the lateral region slits 33b are in closed state so far as the absorbent body 13 is not subjected to a tensile force in the longitudinal direction A. For the absorbent body 13 joined to the backsheet layer 12, the absorbent body 13 is preferably joined to the backsheet layer 12 in the pleat-free regions 27 of the first sheet 23 so that the second sheets 24 may be extended in the longitudinal direction A without any interference. However, it is possible within the scope of the present invention to join the absorbent body 13 to the first sheet 23 in the multi-pleats regions 26 or to join the absorbent body 13 to part of the second sheets 24.

Referring to Fig. 4, the second sheets 24 being adjacent to each other in the longitudinal direction A are distanced from each other so as to keep the pleat-free regions 27 exposed therebetween and, in consequence, these pleat-free regions 27 directly face the absorbent body 13. In this way, the breathability of the first sheet 23 is not affected by the second sheets 24 and serves to let an amount of water vapor generated from the body exudate absorbed by the absorbent body 13 out from the napkin 10.

Fig. 5 is a fragmentary sectional view of the napkin 10 in a state that the napkin 10 has been stretched in the longitudinal direction A with the opposite lateral ends 17 of the napkin 10 illustrated in Fig. 4 pinched by the wearer's fingers. In the napkin 10, the backsheet 12 has an elastic extensibility so that, as illustrated, the respective pleats 25 have been unfolded in the longitudinal direction A as the first sheet 23, the one constituent of the backsheet 12, has been stretched in the longitudinal direction A . Simultaneously, the respective second sheets 24, the other constituents of the backsheet 12, have been elastically stretched. As illustrated, the topsheet 11 joined to the backsheet 12 at the opposite longitudinal sides 16 and the opposite lateral ends 17 has been also stretched in the longitudinal direction A. The arrangement such that the backsheet layer 12 and the side corner sheets 39 are joined together in the compressed recesses 19b formed continually in the longitudinal direction A along the opposite longitudinal sides 16 allows the backsheet 12 and the side corner sheets 39 to be stretched in the longitudinal direction A between each pair of the adjacent compressed recesses 19b. In particular, the arrangement such that the compressed recesses 19b are continually formed in the multi-pleat regions 26 of the backsheet 12 facilitates the multi-pleat regions 26 to be stretched. The absorbent body 13 is joined to the topsheet 11 and/or the backsheet 12 and, in consequence, the absorbent body 13 is opened in the longitudinal direction A at least at the central region slits 33a of the central region slits 33a and the lateral region slits 33b. In this way, the napkin 10 is stretched in the longitudinal direction A. Extensibility of the napkin 10 due to the extensibility of the respective elements is an elastic extensibility based on the presence of the second sheet 24 and, upon being released from the stretching force, the napkin elastically contracts and substantially restores the original state. Hence, the napkin 10 put on the wearer' s body by attaching it to the crotch region of the clothes may elastically expand or contract to conform to the movement of the garment such as torsion or kink due to the movement of the wearer' body. The napkin 10 smoothly expands and contracts in this manner and consequently the attachment regions 35 should not be readily peeled off from the crotch region of the garment. Also in the crotch region, the napkin 10 should not sag and/or only the napkin 10 should not get winkled and, in consequence, the wearer may experience a sense of unity ensured between the napkin 10 and the garment.

In addition, according to this embodiment of the napkin 10, the absorbent body 13 and second sheets are joined together in a manner that all the central region slits 33a of the absorbent body 13 overlap with the first regions 29. With such arrangement, the first regions 29 are elastically expanded as the central region slits 33a of the absorbent body 13 are opened. Furthermore, the backsheet 12 has the first regions 29 extending in the transverse direction B across the absorbent body 13 and expressing the elastic extensibility and the second region 30 in which the elastic extensibility in the longitudinal direction A is lower than that in the first regions 29. With such arrangement, it is possible to make a deformation in the first regions 29 significant and to make a deformation in the second regions 30 insignificant. Whereby, it is possible to subject the napkin 10 to tensile forces opposite to each other in the longitudinal direction A so that the napkin 10 may be sufficiently expanded in the longitudinal direction A. Furthermore, in the napkin 10 according to the present embodiment, the slit-free regions of the absorbent body 13 and the second regions 30 are arranged so as to overlap with each other and joined together so that the deformation of the napkin 10 in the second regions 30 may be further restricted to ensure the above-mentioned effect (sufficient extension of the napkin 10 in the longitudinal direction A ensured by subjecting the napkin 10 to the tensile forces opposite to each other in the longitudinal direction A)

The central region slits 33a arranged in the absorbent body 13 facilitate the napkin 10 to be stretched in the longitudinal direction A merely by pinching regions on the central line P of the opposite lateral ends 17 and pulling the napkin 10 in the longitudinal direction A without being affected by the backsheet 12 and the side corner sheets 39 joined together in the respective longitudinal sides 16. In view of this, the users may recognize this advantageous property merely by pinching the lateral ends 17 and pulling the napkin in the longitudinal direction A and select the napkin 10 according to the present invention without hesitation from various types of napkin commercially available.

Referring again to Fig. 1, the longitudinal sides 16 and the wings 20 of the napkin 10 are formed of the water-repellent or liquid-impermeable side corner sheets 39 and the backsheet 12 joined together, for example, with hot melt adhesive. The side corner sheets 39 respectively have regions 40 in which the respective side corner sheets 39 are joined to the skin-facing surface of the backsheet layer 12, for example, with hot melt adhesive (not shown). In this regard, both inside edge portions 40a except the opposite lateral ends 17 of the respective regions 40 are not joined to the topsheet layer 11 as well as to the backsheet 12 so that gaps may be formed between the inside edge portions 40a and the topsheet 11 and body exudates may be received in these gaps. In this way, it is possible to prevent the body exudates from leaking sideways and/or leaching out.

The napkin 10 in which the absorbent body 13 and the backsheet 12 are arranged and joined to each other in the manner that all the central region slits 33a overlap with the first regions 29 has been described above as the napkin 10 according to the first embodiment of the present invention. However, it is possible within the scope of the present invention, though not illustrated, to arrange the absorbent body and the backsheet and to join them to each other in a manner that all the central region slits overlap with the first regions 29 and all the lateral region slits also overlap with the first regions. Here, the absorbent body and the backsheet are arranged and joined to each other in a manner that a region not formed with the central region slits, a region not formed with the lateral region slits and the second regions overlap together.

### <Second Embodiment>

Referring to Fig. 6, a region in which the topsheet 11, the absorbent body 13 and the backsheet 12 overlap in this order is formed with a plurality of dot-like compressed recesses 42 depressed from the topsheet layer 11 toward the backsheet 12 and located from each other. These compressed recesses 42 are formed by embossing, debossing the both sheets 11, 12 and the absorbent body 13 overlapping in this manner, preferably embossing/debossing them, preferably on heating. In each of the compressed recesses 42, the both sheet layers 11, 12 and the absorbent body 13 are integrated as a result of mechanical confounding or thermal bonding. Such compressed recesses 42 are arranged on the second regions 30 overlapping with the regions formed with none of the slits 33a, 33b and not arranged in the first regions 29 in order to reinforce the integration of the both sheets 11, 12 and the absorbent body 13 and to facilitate the absorbent body 13 to be stretched/contracted in the longitudinal direction A. Such arrangement and the effect thereof are preferable in the other embodiments described below.

### <Third Embodiment>

Referring to Fig. 7, a topsheet layer 41 of the napkin 10 according to this embodiment is formed of a liquid-permeable fibrous nonwoven fabric or porous plastic film having no elastic extensibility in the longitudinal direction A but formed with a plurality of slits 43a extending in the transverse direction B so as to be extensible in the longitudinal direction A. Also the side corner sheets 39 are formed with a plurality of slits 43b extending in the transverse direction B.

In this napkin 10, the side corner sheets 39, the topsheet layer 41 and the absorbent body 13 are arranged and joined together in a manner that all the slits 43b of the side corner sheets 39, all the slits 43a of the topsheet layer 41 and all the slits 33a, 33b of the absorbent body 13 overlap with each other. In this napkin 10, the slits 43b of the side corner sheets 39 and the slits 43a of the topsheet 41 facilitate the slits 33a, 33b of the absorbent body 13 to be opened. In this regard, while all the slits 43a, 43b of the topsheet 41 and the side corner sheets 39 preferably overlap with the slits 33a, 33b of the absorbent body 13, at least respective ones of the slits 43a, 43b of the topsheet 41 and the side corner sheets 39 may overlap with the associated slits 33a, 33b of the absorbent body 13 to ensure the desired effect.

### <First example>

Referring to Fig. 8, in the napkin 10 according to a first example, the topsheet 51 (one of the main sheets) is formed of a liquid-permeable fibrous nonwoven fabric and elastically extensible in the longitudinal direction A and contractible from an elastically stretched state. The topsheet 51 has first regions 44 extending across the absorbent body 13 to express an elastic extensibility in the longitudinal direction A and second regions 45 having an elastic extensibility in the longitudinal direction A lower than that of the first regions 44. The second regions 45 also extend across the absorbent body 13 in the transverse direction B. The first regions 44 and the second regions 45 are alternately arranged in the longitudinal direction A. In this regard, a boundary between each pair of the adjacent first and second regions 44, 45 is indicated by a dashed-two dotted line. The first regions 44 and the second regions 45 respectively extend across the absorbent body 13. According to this example, assuming that, for example, a backsheet 52 has not the elastic extensibility and the topsheet 51 has the elastic extendibility, when the napkin 10 is subjected to a stretching force of 700 mN in the longitudinal direction A per a dimension of 25.0 mm in the transverse direction B, a stretch ratio of the napkin 10 is at least in a range of 1.03 to 1.10. Of course, the stress ratio of the napkin 10 may excess 1.10.

The topsheet 51 and the absorbent body 13 are arranged and joint to each other in a manner that the first regions 44 of the topsheet 51 and all the central region slits 33a of the absorbent body 13 overlap with each other. In this regard, the topsheet 51 and the absorbent body 13 may be arranged and joined to each other in a manner that the first regions 44 of the topsheet 51 overlap with at least respective ones of the slits 33a, 33b of the absorbent body 13.

Referring to Fig. 9, the backsheet 52 is formed only of the elastically non-extensible first sheet 23 and includes none of the above-mentioned narrow second sheets 24. The first sheet 23 is formed in its approximately entire area except the opposite lateral ends 17, the wing portions 20 and the midportion in the longitudinal direction A with the pleats 25. A plurality of such pleats 25 make the first sheet 23, i.e., the backsheet layer 52 extensible in the longitudinal direction A. The backsheet 52 has a pair of multi-pleats regions 26 distanced from each other in the longitudinal direction A and the pleat-free region 27 is defined between these two multi-pleats regions . In addition to this pleat-free region 27, the opposite lateral ends 17 respectively define the pleat-free regions 27. The backsheet 52 formed in this manner is inelastically extensible in the longitudinal direction A under the effect of the multi-pleats regions 26.

For this napkin 10, the absorbent body 13 and the topsheet are arranged and joined to each other in a manner that all the central region slits 33a of the absorbent body 13 overlap with the first regions 44 so that the first regions 44 may be elastically stretched along with opening of the central region slits 33a of the absorbent body 13. Further, the topsheet 51 has the first regions 44 extending in the transverse direction B across the absorbent body 13 to express an elastic extensibility and the second regions 45 having an elastic extensibility in the longitudinal direction A lower than that of the first regions 44. With such arrangement, it is possible to make a deformation in the first regions 44 significant and to make a deformation in the second regions 45 insignificant in the longitudinal direction A. In this way, the napkin 10 may be subjected to tensile forces opposite to each other in the longitudinal direction A to stretch the napkin 10 sufficiently in the longitudinal direction A. Furthermore, for this napkin 10, the topsheet 51 and the absorbent body 13 are arranged and joined to each other in a manner that the slit-free regions of the absorbent body 13 and the second regions 45 overlap with each other. Such arrangement makes the deformation of the napkin in the second regions 45 further insignificant so that the above-mentioned effect (the napkin 10 is subjected to a pair of tensile forces opposite in the longitudinal direction A and whereby the napkin 10 is sufficiently stretched in the longitudinal direction A) may be ensured.

While the first sheet 23 formed in its approximately entire area except the opposite lateral ends 17, the wings 20 and the midportion in the longitudinal direction A with the pleats 25 has been exemplarily described above, the first sheet 23 may be formed in its approximately entire area in the longitudinal direction A except the opposite lateral ends 17 with the pleats 25.

### <Fourth Embodiment>

Referring to Figs. 10 and 11, the napkin 10 according to a fourth embodiment has the topsheet 51 in the first example and the backsheet 12 in the first embodiment. Specifically, the topsheet 51 has the first regions 44 and the second regions 45 and the backsheet 12 has the first regions 29 and the second regions 30. A boundary between each pair of the first and second regions 44, 45 being adjacent in the topsheet 51 is indicated by a dashed-two dotted line extending in the transverse direction B.

In this napkin 10, the top- and backsheets 51, 12 and the absorbent body 13 are arranged and joined together in a manner that all the central region slits 33a, the first regions 44 of the topsheet 51 and the first regions 29 of the backsheet 12 overlap with each other.

According to this embodiment also, when the napkin 10 having the central region slits 33a opened under a pair of tensile forces opposite in the longitudinal direction A is released from such tensile forces, the central region slits 33a rapidly restore the initial state under the contractile force (contractile force of the topsheet 51 and the contractile force of the second sheets 24 of the backsheet 12) of the sheets 12, 51 arranged on the skin-facing surface 11a and the garment-facing surface 12a, respectively.

The terms "first" and "second" used in the specification and claims of the present invention are used merely to distinguish the similar elements, similar positions or the other similar items.

### {Reference Signs List}

- 10: absorbent article (napkin)
- 11: topsheet
- 11a: skin-facing surface
- 12: backsheet
- 12a: garment-facing surface
- 13: absorbent body
- 17: opposite lateral ends
- 23: first sheet
- 24: second sheet
- 25: pleats
- 26: multi-pleats regions
- 27: pleat-free regions
- 29: first region
- 30: second region
- 31: lateral edges
- 33a: central region slits
- 33b: lateral region slits
- 35: attachment regions
- 41: topsheet
- 43a: slits
- 44: first region
- 45: second region
- 51: topsheet
- 52: backsheet
- A: longitudinal direction
- B: transverse direction
- P: center line

## Claims

1. A pad-shaped absorbent article (10) having a longitudinal direction (A) and a transverse direction (B), opposite lateral ends (17) and opposite longitudinal sides (16) and including a liquid-permeable topsheet (11) defining a skin-facing surface (11a), a backsheet (12) defining a garment-facing surface (12a) and an absorbent body (13) interposed between the top- and backsheets (11, 12), **characterised in that**:
the topsheet (11), the backsheet (12) and the absorbent body (13) respectively have an extensibility in the longitudinal direction (A);
the extensibility of the backsheet (12) is an elastic extensibility;
the backsheet (12) has first regions (29) extending across the absorbent body (13) in the transverse direction (B) to express an elastic extensibility and second regions (30) having an elastic extensibility in the longitudinal direction (A) lower than that of the first regions (29);
the absorbent body (13) has a plurality of slits (33a, 33b) extending in the transverse direction (B) and expresses an extensibility in the longitudinal direction (A), when the article is subjected to tensile forces opposite in the longitudinal direction so as to open the slits; and
the absorbent body (13) is arranged so that at least one of the slits (33a, 33b) overlaps with the first regions (29),
the first regions (29) in the backsheet (12) are formed with the first regions (29) defined by a non-extensible first sheet (23) formed with multi-pleats regions (26) each defined by a plurality of pleats (25) extending in the transverse direction (B) and lying side-by-side in the longitudinal direction (A) and elastically extensible second sheets (24) lying between the first sheet (23) and the absorbent body (13) and joined to the first sheet (23) so as to extend across the absorbent body (13) in the transverse direction (B) and to extend across the multi-pleats regions (26) in the longitudinal direction (A);
the backsheet (12) is formed with the multi-pleats regions (26) arranged at intervals in the longitudinal direction (A) and the second regions (30), which are non-extensible pleat-free regions (27) defined between each pair of the adjacent multi-pleats regions (26); and
the absorbent body (13) and the backsheet (12) are joined to each other.

2. The absorbent article according to claim 1 wherein:
the topsheet is formed of non-extensible material;
the topsheet has a plurality of slits (43a) extending in the transverse direction and when the article is subjected to a pair of tensile forces opposite in the longitudinal direction, the slits are opened and thereupon an extensibility in the longitudinal direction is expressed; and
at least one of the slits of the topsheet overlaps with the slits of the absorbent body.

3. The absorbent article according to claim 1 or 2 wherein the second sheets are joined to the pleat-free regions.

4. The absorbent article according to any one of claims 1 through 3 wherein the second regions of the backsheet are provided with attachment regions (35) to clothes.

5. The absorbent article according to any one of claims 1 through 4 wherein:
the topsheet has the first region and the second regions; and
at least one of the slits of the absorbent body overlaps with the first region of the topsheet and the first region of the backsheet.

6. The absorbent article according to any one of claims 1 through 5
wherein a stretch ratio of the article is at least in a range of 1.03 to 1.10 when the article is subjected to a tensile force of 700 mN in the longitudinal direction per a dimension of 25.0 mm in the transverse direction when measured in accordance with the measuring method as described in the description.

7. The absorbent article according to any one of claims 1 through 6 wherein:
the absorbent body has both side edges extending in the longitudinal direction and central region slits in a central area as viewed in the transverse direction arranged in a manner that the none of the central region slits extends to at least one of the both side edges; and
the absorbent body has at least two central region slits (33a).

8. The absorbent article according to any one of claims 1 through 6 wherein:
the article has a center line (P) extending in the longitudinal direction so as to bisect a dimension of the article;
the absorbent body has lateral edges (31) opposed to each other in the transverse direction and extending in the longitudinal direction and lateral region slits extending in the transverse direction from the lateral edges toward the center line but not to the center line; and
the absorbent body has at least two lateral region slits (33b) .

## Patentansprüche

1. Einlagen-förmiger absorbierender Artikel (10), der Folgendes aufweist: eine Längsrichtung (A) und eine Querrichtung (B), gegenüberliegende seitliche Enden (17) und gegenüberliegende Längsseiten (16), und der Folgendes einschließt: eine flüssigkeitsdurchlässige obere Lage (11), die eine der Haut zugewandte Oberfläche (11a) definiert, eine untere Lage (12), die eine der Bekleidung zugewandte Oberfläche (12a) definiert, und einen Saugkörper (13), der zwischen der oberen und unteren Lage (11, 12) eingefügt ist, **dadurch gekennzeichnet, dass**:
die obere Lage (11), die untere Lage (12) und der Saugkörper (13) jeweils eine Dehnbarkeit in der Längsrichtung (A) aufweisen;
die Dehnbarkeit der unteren Lage (12) eine elastische Dehnbarkeit ist;
die untere Lage (12) Folgendes aufweist: erste Bereiche (29), die sich über den Saugkörper (13) in der Querrichtung (B) erstrecken, um eine elastische Dehnbarkeit wiederzugeben, und zweite Bereiche (30), die eine elastische Dehnbarkeit in der Längsrichtung (A) aufweisen, die geringer ist als die der ersten Bereiche (29);
der Saugkörper (13) eine Vielzahl von Schlitzen (33a, 33b) aufweist, die sich in der Querrichtung (B) erstrecken, und eine Dehnbarkeit in der Längsrichtung (A) wiedergibt, wenn der Artikel in der Längsrichtung entgegengesetzten Zugkräften ausgesetzt wird, sodass sich die Schlitze öffnen; und
der Saugkörper (13) derart angeordnet ist, dass mindestens einer der Schlitze (33a, 33b) mit den ersten Bereichen (29) überlappt,
die ersten Bereiche (29) in der unteren Lage (12) mit den ersten Bereichen (29) ausgebildet sind, die definiert sind durch: eine nichtdehnbare erste Lage (23), die mit Bereichen mit vielen Falten (26) ausgebildet ist, wobei jeder durch eine Vielzahl von Falten (25) definiert ist, die sich in der Querrichtung (B) erstrecken und nebeneinander in der Längsrichtung (A) vorliegen, und elastisch dehnbare zweite Lagen (24), die zwischen der ersten Lage (23) und dem Saugkörper (13) vorliegen und mit der ersten Lage (23) verbunden sind, sodass sie sich über den Saugkörper (13) in der Querrichtung (B) erstrecken und sich über die Bereiche mit vielen Falten (26) in der Längsrichtung (A) erstrecken;
die untere Lage (12) mit den Bereichen mit vielen Falten (26), die in Abständen in der Längsrichtung (A) angeordnet sind, und den zweiten Bereichen (30), die nichtdehnbare Bereiche ohne Falten (27) sind, die zwischen jedem Paar der benachbarten Bereiche mit vielen Falten (26) definiert sind, ausgebildet ist; und
der Saugkörper (13) und die untere Lage (12) miteinander verbunden sind.

2. Absorbierender Artikel nach Anspruch 1, wobei:
die obere Lage aus nichtdehnbarem Material ausgebildet ist;
die obere Lage eine Vielzahl von Schlitzen (43a) aufweist, die sich in der Querrichtung erstrecken, und, wenn der Artikel einem Paar von in der Längsrichtung entgegengesetzten Zugkräften ausgesetzt ist, die Schlitze geöffnet werden und dabei eine Dehnbarkeit in der Längsrichtung wiedergegeben wird; und
mindestens einer der Schlitze der oberen Lage mit den Schlitzen des Saugkörpers überlappt.

3. Absorbierender Artikel nach Anspruch 1 oder 2, wobei die zweiten Lagen mit den Bereichen ohne Falten verbunden sind.

4. Absorbierender Artikel nach einem der Ansprüche 1 bis 3, wobei die zweiten Bereiche der unteren Lage mit Bereichen zur Befestigung (35) an Kleidung bereitgestellt sind.

5. Absorbierender Artikel nach einem der Ansprüche 1 bis 4, wobei:
die obere Lage den ersten Bereich und die zweiten Bereiche aufweist; und
mindestens einer der Schlitze des Saugkörpers mit dem ersten Bereich der oberen Lage und dem ersten Bereich der unteren Lage überlappt.

6. Absorbierender Artikel nach einem der Ansprüche 1 bis 5,
wobei ein Streckverhältnis des Artikels mindestens in einem Bereich von 1,03 bis 1,10 liegt, wenn der Artikel einer Zugkraft von 700 mN in der Längsrichtung pro Abmessung von 25,0 mm in der Querrichtung, bei Messung gemäß dem Messverfahren wie in der Beschreibung beschrieben, ausgesetzt wird.

7. Absorbierender Artikel nach einem der Ansprüche 1 bis 6, wobei:
der Saugkörper Folgendes aufweist: beide Seitenränder, die sich in der Längsrichtung erstrecken, und Schlitze des mittleren Bereichs in einer mittleren Fläche, bei Betrachtung in der Querrichtung, in einer Weise angeordnet, dass sich keiner der Schlitze des mittleren Bereichs zu mindestens einem der beiden Seitenränder erstreckt; und
der Saugkörper mindestens zwei Schlitze des mittleren Bereichs (33a) aufweist.

8. Absorbierender Artikel nach einem der Ansprüche 1 bis 6, wobei:
der Artikel eine Mittellinie (P) aufweist, die sich in der Längsrichtung erstreckt, sodass sie eine Abmessung des Artikels halbiert;
der Saugkörper Folgendes aufweist: Seitenränder (31), die einander in der Querrichtung gegenüberliegen und sich in der Längsrichtung erstrecken, und Schlitze des seitlichen Bereichs, die sich in der Querrichtung von den Seitenrändern in Richtung der Mittellinie, doch nicht bis zur Mittellinie erstrecken; und
der Saugkörper mindestens zwei Schlitze des seitlichen Bereichs (33b) aufweist.

## Revendications

1. Article absorbant en forme de serviette hygiénique (10) ayant une direction allant dans le sens longitudinal (A) et une direction allant dans le sens transversal (B), des extrémités latérales opposées (17) et des côtés longitudinaux opposés (16) et comprenant une feuille supérieure perméable aux liquides (11) définissant une surface orientée vers la peau (11a), une feuille de support (12) définissant une surface orientée vers le vêtement (12a) et un corps absorbant (13) intercalé entre les feuilles supérieure et de support (11, 12), **caractérisé en ce que** :
la feuille supérieure (11), la feuille de support (12) et le corps absorbant (13) ont respectivement une extensibilité dans la direction allant dans le sens longitudinal (A) ;
l'extensibilité de la feuille de support (12) est une extensibilité élastique ;
la feuille de support (12) a des premières régions (29) s'étendant en travers du corps absorbant (13) dans la direction allant dans le sens transversal (B) pour exprimer une extensibilité élastique et des deuxièmes régions (30) ayant une extensibilité élastique dans la direction allant dans le sens longitudinal (A) inférieure à celle des premières régions (29) ;
le corps absorbant (13) a une pluralité de fentes (33a, 33b) s'étendant dans la direction allant dans le sens transversal (B) et exprime une extensibilité dans la direction allant dans le sens longitudinal (A), quand l'article est soumis à des forces de traction opposées dans la direction allant dans le sens longitudinal de manière à ouvrir les fentes ; et
le corps absorbant (13) est agencé de telle sorte qu'au moins l'une des fentes (33a, 33b) chevauche les premières régions (29),
les premières régions (29) dans la feuille de support (12) sont formées avec les premières régions (29) définies par une première feuille non extensible (23) formée avec des régions à plis multiples (26) définies chacune par une pluralité de plis (25) s'étendant dans la direction allant dans le sens transversal (B) et reposant côte à côte dans la direction allant dans le sens longitudinal (A) et des deuxièmes feuilles extensibles de manière élastique (24) reposant entre la première feuille (23) et le corps absorbant (13) et assemblées sur la première feuille (23) de manière à s'étendre en travers du corps absorbant (13) dans la direction allant dans le sens transversal (B) et à s'étendre en travers des régions à plis multiples (26) dans la direction allant dans le sens longitudinal (A) ;
la feuille de support (12) est formée avec les régions à plis multiples (26) agencées selon des intervalles dans la direction allant dans le sens longitudinal (A) et les deuxièmes régions (30), qui sont des régions non extensibles exemptes de plis (27) définies entre chaque paire des régions adjacentes à plis multiples (26) ; et
le corps absorbant (13) et la feuille de support (12) sont assemblés l'un sur l'autre.

2. Article absorbant selon la revendication 1, dans lequel :
la feuille supérieure est formée à partir d'un matériau non extensible ;
la feuille supérieure a une pluralité de fentes (43a) s'étendant dans la direction allant dans le sens transversal et quand l'article est soumis à une paire de forces de traction opposées dans la direction allant dans le sens longitudinal, les fentes sont ouvertes et sur ce une extensibilité dans la direction allant dans le sens longitudinal est exprimée ; et
au moins l'une des fentes de la feuille supérieure chevauche les fentes du corps absorbant.

3. Article absorbant selon la revendication 1 ou la revendication 2, dans lequel les deuxièmes feuilles sont assemblées sur les régions exemptes de plis.

4. Article absorbant selon l'une quelconque des revendications 1 à 3, dans lequel les deuxièmes régions de la feuille de support comportent des régions d'attache (35) aux vêtements.

5. Article absorbant selon l'une quelconque des revendications 1 à 4, dans lequel :
la feuille supérieure a la première région et les deuxièmes régions ; et
au moins l'une des fentes du corps absorbant chevauche la première région de la feuille supérieure et la première région de la feuille de support.

6. Article absorbant selon l'une quelconque des revendications 1 à 5, dans lequel un rapport d'étirage de l'article se trouve au moins dans une plage allant de 1,03 à 1,10 quand l'article est soumis à une force de traction de 700 mN dans la direction allant dans le sens longitudinal par dimension de 25,0 mm dans la direction allant dans le sens transversal quand la mesure est effectuée en fonction du procédé de mesure décrit dans la description.

7. Article absorbant selon l'une quelconque des revendications 1 à 6, dans lequel :
le corps absorbant a les deux bords latéraux qui s'étendent dans la direction allant dans le sens longitudinal et les fentes de la région centrale dans une zone centrale quand vue dans la direction allant dans le sens transversal dont l'agencement est tel que ladite aucune des fentes de la région centrale ne s'étend jusqu'à au moins l'un des deux bords latéraux ; et
le corps absorbant a au moins deux fentes de la région centrale (33a).

8. Article absorbant selon l'une quelconque des revendications 1 à 6, dans lequel :
l'article a une ligne centrale (P) s'étendant dans la direction allant dans le sens longitudinal de manière à croiser une dimension de l'article ;
le corps absorbant a des bords latéraux (31) opposés l'un par rapport à l'autre dans la direction allant dans le sens transversal et s'étendant dans la direction allant dans le sens longitudinal et des fentes de la région latérale s'étendant dans la direction allant dans le sens transversal depuis les bords latéraux vers la ligne centrale mais non pas jusque sur la ligne centrale ; et
le corps absorbant a au moins deux fentes de la région latérale (33b).
